(19) Europäisches Patentamt
European Patent Office
Office européen des brevets

(11) Publication number : 0 431 942 B1

(12) # EUROPEAN PATENT SPECIFICATION

(45) Date of publication of patent specification : **23.03.94 Bulletin 94/12**

(51) Int. Cl.$^5$ : **A61K 31/135,** A61K 31/22, A61K 31/455, A61K 47/14

(21) Application number : **90313252.0**

(22) Date of filing : **06.12.90**

(54) **Percutaneously absorbable pharmaceutical preparation containing dopamine derivative.**

The file contains technical information submitted after the application was filed and not included in this specification

(30) Priority : **06.12.89 JP 317343/89**
**20.06.90 JP 161845/89**

(43) Date of publication of application :
**12.06.91 Bulletin 91/24**

(45) Publication of the grant of the patent :
**23.03.94 Bulletin 94/12**

(84) Designated Contracting States :
**DE FR GB IT**

(56) References cited :
**EP-A- 0 279 986**
**EP-A- 0 321 870**
**GB-A- 2 141 025**
**GB-A- 2 142 237**
**Chem. Pharm. Bull. 37(5), 1375-1378 (1989)**
**Römpp's Chemie-Lexikon, 8th ed., Franck-'sche Verlagsbuchhandlung, Stuttgart, 3428 (1987)**
**Römpp's Chemie-Lexikon, 9th ed., 702 (1989)**

(73) Proprietor : **SANSHO CO., LTD.**
**3-5-4, Hongo**
**Bunkyo-ku, Tokyo (JP)**
Proprietor : **Takasago International Corporation**
**19-22, Takanawa 3-chome Minato-ku**
**Tokyo (JP)**

(72) Inventor : **Yoshida, Mitsuhiro**
**Wariyama-Haitsu 201, 2924-3, Uwanodai**
**Fukaya-shi, Saitama (JP)**
Inventor : **Fujimori, Hiroyuki**
**Sansho-Ryo 2-4, 1034-1, Imai**
**Honjo-shi, Saitama (JP)**
Inventor : **Ishino, Yoshio**
**730-53, Kumagaya**
**Kumagaya-shi, Saitama (JP)**
Inventor : **Sasaki, Akira**
**3-17-17-2, Shakujiidai**
**Nerima-ku, Tokyo (JP)**
Inventor : **Kasai, Masayoshi**
**5-156, Tsutsujigaoka**
**Kagamihara--shi, Gifu (JP)**
Inventor : **Ohmori, Keiji**
**1185-2, Itai, Tamamura-machi**
**Sawa-gun, Gunma (JP)**
Inventor : **Konita, Noriko**
**3-2-2, Honcho, Fukiage-machi**
**Kitaadachi-gun, Saitama (JP)**
Inventor : **Yuasa, Yoshifumi, c/o Takasago Int. Corp.**
**Kamata Div., 36-31, Kamata 5-chome**
**Ohta-ku, Tokyo (JP)**
Inventor : **Kobayashi, Toyohiko, c/o Takasago Int. Corp.**
**Kamata Div., 36-31, Kamata 5-chome**
**Ohta-ku, Tokyo (JP)**

(74) Representative : **Diamond, Bryan Clive et al**
**Gee & Co. Chancery House Chancery Lane**
**London WC2A 1QU (GB)**

EP 0 431 942 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention relates to a percutaneously absorbed pharmaceutical containing a dopamine derivative which preparation has a high permeability through the skin and a low skin irritativeness.

Dopamine, which is represented by formula (II):

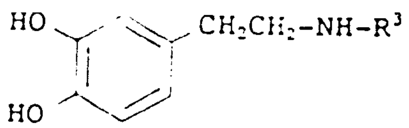

(II)

wherein $R^3$ is a hydrogen atom;
and dobutamine, which is represented by the above formula (II) wherein $R^3$ is a group,

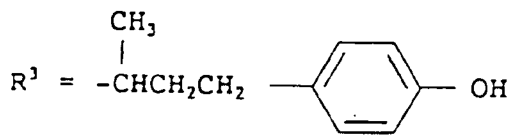

,

have been used for the treatment of, for example, hypotension, cardiac circulatory insufficiency and cardinogenic shock. However, since each of these compounds has a free catechol group and is scarcely absorbed when administered orally or percutaneously, their administration has been restricted to the continuous intravenous route.

Recently, ibopamine represented by formula (III):

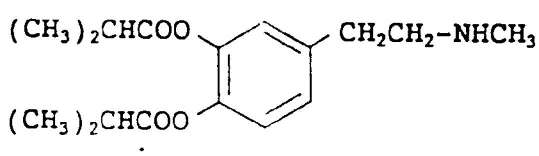

(III)

has been developed as a drug which exerts a dopamine-like effect and can be orally administered (cf. U.S. Patents 4,218,470 and 4,302,471). However this compound is still unsatisfactory in sustained effect and it is reported that the half-life of this compound in plasma is 1.54 hour [cf. E. Lodola et al; Arzneim.-Forsch./Drug Res., 36(1), 345-348 (1986)]. It is further reported that the effect of ibopamine on improvement of the circulation, when evaluated by the cardiac coefficient, sustains 3 to 6 hours [cf. SOL I. Rajifer et al.; Circulation, 73, 740-748 (1986)].

Recently, further attempts have been made to develop a preparation capable of gradually releasing a drug in the blood for a prolonged period of time so as to improve the persistence of the effect of the drug. For example, U.S. Patent 4,581,225 discloses a sustained release nasal preparation which comprises a catechol amine (for example, dobutamine), an emulsifier, a dispersant and an agent rendering the drug slowly released such as oleic acid, linolic acid or an ester thereof. Further, Okumura et al. have studied on the application of dopamine hydrochloride to the skin and reported that the use of a 5% aqueous solution of glyceryl monocaprylate can elevate the percutaneous penetration rate of dopamine hydrochloride to a level 34 times as high as that achieved by using dopamine hydrochloride alone [cf. Chem. Pharm. Bull., 37(5), 1375-1378 (1989)]. In the practical clinical application, however, it is preferable to establish a higher skin permeability. There is an additional problem, furthermore, that dopamine hydrochloride is highly irritative to the skin and causes skin damage if it can be percutaneously absorbed. Therefore, this compound is unsuitable for practical application.

Other known dopamine derivatives, which might exert the dopamine-like effects, include those synthesized by introducing various substituents into the catechol and/or amino group [cf. C. Casagrande et al.; Arzneim.-Forsch./Drug Res., 36(2), 291-303 (1986)].

An object of the present invention is to provide a percutaneously absorbable preparation having dopamine-like effects, which is expected to be prolonged for a long period of time, a high skin permeability and a low skin irritativeness. We have now found that the use of lactic acid esters, or higher alcohols in combination with

a dopamine derivative represented by the formula (I) greatly promote the percutaneous absorption of said compound; and that the use of an unsaturated fatty acid monohydric alcohol ester remarkably reduces the skin irritativeness of said compound.

Accordingly, the present invention provides a percutaneously absorbable pharmaceutical preparation which comprises the following ingredients (A) and (B):

(A) a diacylated dopamine derivative represented by formula (I):

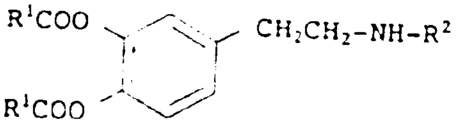

(I)

wherein $R^1$ represents an alkyl group having 3 to 7 carbon atoms, a cycloalkyl group having 3 to 6 carbon atoms, a substituted or unsubstituted phenyl group or a substituted or unsubstituted nitrogen-containing heterocylic group; and $R^2$ represents a hydrogen atom or an alkyl group having up to 4 carbon atoms; or a salt thereof;and

(B) one or more compounds selected from lactic acid esters selected from myristyl lactate, cetyl lactate and lauryl lactate, and a saturated or unsaturated alcohol having 12 to 18 carbon atoms.

The pharmaceutical preparation may additionally contain (C) an unsaturated fatty acid monohydric alcohol ester.

In formula (I), the two $R^1$ both represent the same group. Examples of the diacylated derivative of dopamine or a salt thereof to be used in the present invention as the ingredient (A) include 3,4-di-O-n-butyryldopamine, 3,4-di-O-n-butyrylepinine (epinine means N-methyldopamine), 3,4-di-O-n-butyryl-N-ethyldopamine, 3,4-di-O-n-butyryl-N-n-propyldopamine, 3,4-di-O-n-butyryl-N-isopropyldopamine, 3,4-di-O-isobutyryldopamine, 3,4-di-O-isobutyrylepinine, 3,4-di-O-isobutyryl-N-ethyldopamine, 3,4-di-O-isobutyryl-N-n-propyldopamine, 3,4-di-O-isobutyryl-N-isopropyldopamine, 3,4-di-O-pivaloyldopamine, 3,4-di-O-pivaloylepinine, 3,4-di-O-pivaloyl-N-ethyldopamine, 3,4-di-O-pivaloyl-N-n-propyldopamine, 3,4-di-O-pivaloyl-N-isopropyldopamine, 3,4-di-O-valeryldopamine, 3,4-di-O-valerylepinine, 3,4-di-O-valeryl-N-ethyldopamine, 3,4-di-O-valeryl-N-n-propyldopamine, 3,4-di-O-valeryl-N-isopropyldopamine, 3,4-di-O-isovaleryldopamine, 3,4-di-O-isovalerylepinine, 3,4,-di-O-isovaleryl-N-ethyledopamine, 3,4,-di-O-isovaleryl-N-n-propyldopamine, 3,4-di-O-isovaleryl-N-isopropyldopamine, 3,4-di-O-caproyldopamine, 3,4-di-O-caproylepinine, 3,4-di-O-caproyl-N-ethyldopamine, 3,4-di-O-caproyl-N-n-propyldopamine, 3,4-di-O-caproyl-N-isopropyldopamine, 3,4-di-O-2'-ethylbutyryldopamine, 3,4-di-O-2'-ethylbutyrylepinine, 3,4-di-O-2'-ethylbutyryl-N-ethyldopamine, 3,4-di-O-2'-ethylbutyryl-N-n-propyldopamine, 3,4-di-O-2'-ethylbutyryl-N-isopropyldopamine, 3,4-di-O-2',2'-dimethylbutyryldopamine, 3,4-di-O-2',2'-dimethylbutyrylepinine, 3,4-di-O-2',2'-dimethylbutyryl-N-ethyldopamine, 3,4-di-O-2',2'-dimethylbutyryl-N-n-propyldopamine, 3,4-di-O-2',2'-dimethylbutyryl-N-isopropyldopamine, 3,4-di-O-3',3'-dimethylbutyryldopamine, 3,4-di-O-3',3'-dimethylbutyrylepinine, 3,4-di-O-3',3'-dimethylbutyryl-N-ethyldopamine, 3,4-di-O-3',3'-dimethylbutyryl-N-n-propyldopamine, 3,4-di-O-3',3'-dimethylbutyryl-N-isopropyldopamine, 3,4-di-O-heptanoyldopamine, 3,4-di-O-heptanoylepinine, 3,4-di-O-heptanoyl-N-ethyldopamine, 3,4-di-O-heptanoyl-N-n-propyldopamine, 3,4-di-O-heptanoyl-N-isopropyldopamine, 3,4-di-O-cyclopropylcarbonyldopamine, 3,4-di-O-cyclopropylcarbonylepinine, 3,4-di-O-cyclopropylcarbonyl-N-ethyldopamine, 3,4-di-O-cyclopropylcarbonyl-N-n-propyldopamine, 3,4-di-O-cyclopropylcarbonyl-N-isopropyldopamine, 3,4-di-O-cyclobutylcarbonyldopamine, 3,4-di-O-cyclobutylcarbonylepinine, 3,4-di-O-cyclobutylcarbonyl-N-ethyldopamine, 3,4-di-O-cyclobutylcarbonyl-N-n-propyldopamine, 3,4-di-O-cyclobutylcarbonyl-N-isopropyldopamine, 3,4-di-O-cyclopentylcarbonyldopamine, 3,4-di-O-cyclopentylcarbonylepinine, 3,4-di-O-cyclopentylcarbonyl-N-ethyldopamine, 3,4-di-O-cyclopentylcarbonyl-N-n-propyldopamine, 3,4-di-O-cyclopentylcarbonyl-N-isopropyldopamine, 3,4-di-O-cyclohexylcarbonyldopamine, 3,4-di-O-cyclohexylcarbonylepinine, 3,4-di-O-cyclohexylcarbonyl-N-ethyldopamine, 3,4-di-O-cyclohexylcarbonyl-N-n-propyldopamine, 3,4-di-O-cyclohexylcarbonyl-N-isopropyldopamine, 3,4-di-O-benzoyldopamine, 3,4-di-O-benzoylepinine, 3,4-di-O-benzoyl-N-ethyldopamine, 3,4-di-O-benzoyl-N-n-propyldopamine, 3,4-di-O-benzoyl-N-isopropyldopamine, 3,4-di-O-4'-methylbenzoyldopamine, 3,4-di-O-4'-methylbenzoylepinine, 3,4-di-O-4'-methylbenzoyl-N-ethyldopamine, 3,4-di-O-4'-methylbenzoyl-N-n-propyldopamine, 3,4-di-O-4'-methylbenzoyl-N-isopropyldopamine, 3,4-di-O-4'-methoxybenzoyldopamine, 3,4-di-O-4'-methoxybenzoylepinine, 3,4-di-O-4'-methoxybenzoyl-N-ethyldopamine, 3,4-di-O-4'-methoxybenzoyl-N-n-propyldopamine, 3,4-di-O-4'-methoxybenzoyl-N-isopropyldopamine, 3,4-di-O-2'-pyrrolinoyldopamine, 3,4-di-O-2'-pyrrolinoylepinine, 3,4-di-O-2'-pyrrolinoyl-N-ethyldopamine, 3,4-di-O-2'-pyrrolinoyl-N-n-propyldopamine, 3,4-di-O-2'-pyrrolinoyl-N-isopropyldopamine, 3,4-di-O-4'-pyrazonoyldopamine, 3,4-di-O-4'-pyrazonoylepinine, 3,4-di-O-4'-pyrazonoyl-N-ethyldopamine, 3,4-di-O-4'-pyrazonoyl-N-n-propyldopamine, 3,4-di-O-4'-pyrazonoyl-N-isopropyldopamine, 3,4-di-O-nicotinoyldopamine, 3,4-di-O-nicotinoylepinine, 3,4-di-O-nicotinoyl-N-ethyldopamine, 3,4-di-O-nicotinoyl-N-n-propyldopamine, 3,4-di-O-nicotinoyl-N-isopropyldopamine, 3,4-di-O-5'-bromonicotinoyldopamine, 3,4-di-O-5'-bromonicotinoylepinine, 3,4-di-O-5'-bromonicotinoyl-N-ethyldopamine, 3,4-di-O-5'-bromonicotinoyl-N-n-propyldopamine, 3,4-di-O-5'-bromonicotinoyl-N-isopropyldopamine, 3,4-di-O-2'-methylnicotinoyldopamine, 3,4-di-O-2'-methylnicotinoylepinine, 3,4-di-O-2'-methylnicotinoyl-N-ethyldopamine, 3,4-di-O-2'-methylnicotinoyl-N-n-propyldopamine, 3,4-di-O-2'-methylnicotinoyl-N-isopropyldopamine, 3,4-di-O-6'-methylnicotinoyldopamine, 3,4-di-O-6'-methylnicotinoyl-epinine, 3,4-di-O-6'-methylnicotinoyl-N-ethyldopamine, 3,4-di-O-6'-methylnicotinoyl-N-n-propyldopamine, 3,4-di-O-6'-methylnicotinoyl-N-isopropyldopamine, 3,4-di-O-2'-pyrazinecarbonyldopamine, 3,4-di-O-2'-pyrazinecarbonylepinine, 3,4-di-O-2'-pyrazinecarbonyl-N-ethyldopamine, 3,4-di-O-2'-pyrazinecarbonyl-N-n-propyldopamine, 3,4-di-O-2'-pyrazinecarbonyl-N-isopropyldopamine and nontoxic organic or inorganic acid salts thereof.

Examples of the organic acid salts include maleates, succinates and tartrates. Examples of the inorganic acid salts include hydrochlorides, hydrobromates, phosphates, nitrates and sulfates.

In formula (I), $R^1$ preferably represents a branched alkyl group or a pyridyl group and $R^2$ preferably represents a hydrogen atom and a methyl group. Among the compounds represented by formula (I) (hereinafter referred to as "Compound (I)"; the same expression shall apply to the other compound), 3,4-di-O-isobutyryldopamine, 3,4-di-O-pivaloyldopamine, 3,4-di-O-isobutyrylepinine and 3,4-di-O-nicotinoyldopamine, and hydrochlorides thereof are particularly preferred.

These dopamine derivatives and salts thereof may be synthesized by known methods with the use of dopamine or epinine as a starting material [cf. R.B. Walker et al.; J. Pharm. Sci., 67, 558 (1978), C. Casagrande et al.; Farmaco Ed. Sci., 28, 143 (1973) and C. Casagrande et al.; Arzneim.-Forsch./Drug Res., 36(2), 291-303 (1986)]. For example, 3,4-di-O-pivaloyldopamine represented by the following formula (I') may be synthesized in accordance with the following reaction scheme.

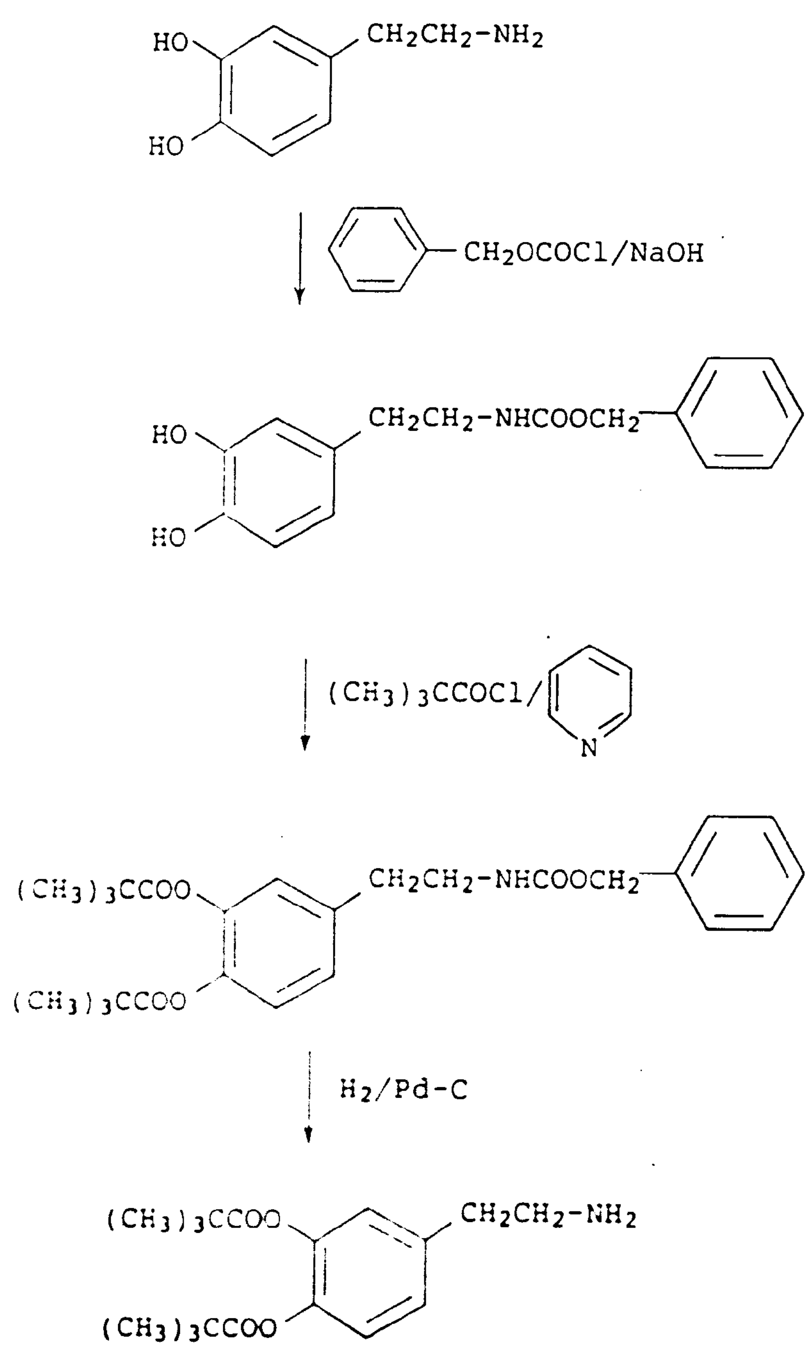


(I'-a)

(I'-b)

(I')

Namely, dopamine is reacted with benzyl chlorocarbonate in the presence of an alkali such as sodium hydroxide to protect the amino group. The intermediate (I'-a) thus obtained is then reacted with an acylating agent. Examples of the acylating agent include acyl anhydride, acyl chloride and acyl bromide. For example, pivaloyl chloride is reacted with the intermediate in pyridine to give a compound of the formula (I'-b). The compound (I'-b) is then subjected to catalytic reduction in methanol or acetic acid with the use of a metal catalyst (for example, palladium black, palladium carbon, Raney nickel, preferably palladium carbon) to give the desired compound (I'). Further, the thus obtained compound (I') may be treated with an appropriate organic or inorganic acid selected from among those cited above and thus a corresponding salt thereof can be obtained.

The content of the dopamine derivative or a salt thereof, namely, the ingredient (A) in the preparation of the present invention ranges from 1 to 20% by weight, preferably 5 to 10% by weight, based on the weight of the preparation.

The lactic acid esters in the ingredient (B) to be used in the preparation of the present invention are myristyl

lactate, cetyl lactate and lauryl lactate. Preferred examples of the C12-18 alcohols include lauryl alcohol, myristyl alcohol and oleyl alcohol.

As the ingredient (B), either one compound selected from lactic acid esters and C12-18 alcohols or a mixture of two or more thereof may be employed. Preferably, lactic acid esters are used. In the preparation of the present invention, the proportion of the ingredient (B) contained in total to the dopamine derivative or a salt thereof ranges from 0.1 to 5, preferably 0.5 to 3, by weight.

A preparation of the present invention may further contain an unsaturated fatty acid monohydric alcohol ester, designated as ingredient (C), in order to reduce skin irritativeness. Examples of said unsaturated fatty acid monohydric alcohol ester include methyl linoleate, ethyl linoleate, propyl linoleate, methyl oleate, ethyl oleate and methyl linolenate. Among those compounds, methyl linoleate and ethyl linoleate are particularly preferable.

The proportion of such unsaturated fatty acid monohydric alcohol ester, if present (ingredient (C)) to the dopamine derivative or salt thereof ranges from 0.1 to 10, preferably 0.3 to 5, by weight.

The form of the preparation of the present invention is not particularly restricted. It may be formulated into, for example an ointment, cream, gel, plaster or taping preparation.

In the preparation of the present invention, any base material may be used so long as it is commonly used in percutaneous preparations. Oily base materials and aqueous base materials may be used therefor. Examples of oily base materials include white petroleum jelly, purified lanolin, squalane, silicone, liquid paraffin, vegetable oils and waxes. Examples of aqueous base materials include water, lower alcohols, polyhydric alcohols and water-soluble polymers. Furthermore, base materials commonly employed in plasters may be used therefor. Examples of these base materials include sticky materials such as natural rubber, synthetic rubber, a styrene/isoprene/styrene copolymer (hereinafter referred to as SIS rubber), polyacrylate resins and polyisobutylene resins and polymer compositions such as soft polyamide resins, polyvinyl alcohol and polyacryl resins.

In addition to the above-described ingredients, the preparation of the present invention may further contain appropriate additives which are commonly employed in percutaneous preparations, for example, surfactants, stabilizers, preservatives (e.g,, methyl benzoate, ethyl benzoate and propyl benzoate) and antiseptics (e.g., benzoic acid).

Usable as a substrate for plaster or taping preparation are laminated films of polyethylene terephthalate and polyethylene vinyl alcohol.

The preparation of the present invention is applied onto the chest, the back or an arm of a patient usually once a day for treating, for example, hypotension or circulatory insufficiency.

The percutaneous preparation of the present invention has the following characteristics which make it useful as a remedy for, for example, hypotension or cardiac circulatory insufficiency.

(1) It suffers from no first-pass effect in the liver and thus can be effectively utilized.

(2) It causes no damage to digestive tracts.

(3) It has a prolonged efficiency.

(4) It has little skin irritativeness.

(5) When any side effect (for example, hypersensitiveness such as eruption) is observed, the administration thereof can be immediately ceased, which means that it has a high safety.

The results of testing the skin permeability and skin irritativeness of the preparation of the present invention will be given with reference to the following test examples and to the accompanying drawings, wherein:

Figs. 1 to 8 each show the results of the determination of the accumulated amount of the drug permeated through human skin with lapse of time of test samples in the in vitro skin permeation tests performed in Test Examples 1 to 8.

TEST EXAMPLES

(1) in vitro skin permeability

(i) Preparation of sample

Test on skin permeability upon the use of lactic acid esters [the ingredient (B)]

Comparative samples:

100 mg of each dopamine derivative hydrochloride as shown in Table 1 was weighed and 900 mg of propylene glycol was added thereto to obtain a solution or suspension containing 10% by weight of each dopamine derivative hydrochloride. These solutions and suspensions were referred to as comparative samples a to e,

respectively.

Invention samples:

100 mg of each dopamine derivative hydrochloride as shown in Table 1 was weighed. Then 50 mg (in the cases of invention samples A to C) or 100 mg (in the cases of invention samples E to G) of each lactic acid esters as shown in Table 1 was added thereto. Further, propylene glycol was added to make the total amount 1000 mg. Thus a solution or suspension containing 10% by weight of each dopamine derivative hydrochloride was obtained. These solutions and suspensions were referred to as invention samples A, B, C, E and G.

Table 1

| Test Ex. | Sample lot | Sample No. | Ingredient: Dopamine derivative | Ingredient: Lactic acid ester or |
|---|---|---|---|---|
| 1 | Comp. | a | 3,4-di-O-pivaloyldopamine hydrochloride | |
| | Comp. | b | 3,4-di-O-isobutyryldopamine hydrochloride | - |
| | Invent. | A | 3,4-di-O-pivaloyldopamine hydrochloride | myristyl lactate |
| | Invent. | B | 3,4-di-O-isobutyryldopamine hydrochloride | cetyl lactate |
| 2 | Comp. | c | 3,4-di-O-isobutyrylepinine hydrochloride | - |
| | Invent. | C | 3,4-di-O-isobutyrylepinine hydrochloride | myristyl lactate |
| 3 | Comp. | d | 3,4-di-O-nicotinoyldopaimne hydrochloride | - |
| | Invent. | E | 3,4-di-O-nicotinoyldopaimne hydrochloride | lauryl lactate |
| 4 | Comp. | e | 3,4-di-O-n-butyryldopamine hydrochloride | - |
| | Ivent. | G | 3,4-di-O-n-butyryldopamine hydrochloride | cetyl lactate |

Test on skin permeability upon the use of higher alcohol [the ingredient (B)] and test on skin permeability upon the combination use of higher alcohol [the ingredient (B)] and unsaturated fatty acid monohydric alcohol ester [the ingredient (C)]

Comparative samples:

100 mg of each dopamine derivative hydrochloride as shown in Table 2 was weighed and 900 mg of polyethylene glycol 400 was added thereto. Thus a solution or suspension containing 10% by weight of each dopamine derivative hydrochloride was obtained. These solutions and suspensions were referred to as comparative samples f to i respectively.

Invention samples:

100 mg of each dopamine derivative hydrochloride as shown in Table 2 was weighed and 100 mg of each higher alcohol as shown in Table 2 (in the cases of invention samples I, K, M and N) or 100 mg of each higher alcohol and 100 mg of each unsaturated fatty acid monohydric alcohol ester (in the cases of invention samples J and L) were added. Further polyethylene alcohol was added thereto to make the total amount 1000 mg. Thus a solution or suspension containing 10% by weight of each dopamine derivative hydrochloride was prepared. These solutions and suspensions were referred to as invention samples I to N, respectively.

Table 2

| Test Ex. | Sample lot | Sample No. | Ingredient | | |
|---|---|---|---|---|---|
| | | | Dopamine derivative | Higher alcohol | Unsaturated fatty acid monoglyceride alcohol ester |
| 5 | Comp. | f | 3,4-di-O-pivaloyl-dopamine hydrochloride | - | - |
| | Invent. | I | 3,4-di-O-pivaloyl-dopamine hydrochloride | oleyl alcohol | - |
| | Invent. | J | 3,4-di-O-pivaloyl-dopamine hydrochloride | oleyl alcohol | ethyl linoleate |
| 6 | Comp. | g | 3,4-di-O-isobutyryl-dopamine hydrochloride | - | - |
| | Invent. | K | 3,4-di-O-isobutyryl-dopamine hydrochloride | myristyl alcohol | - |
| | Invent. | L | 3,4-di-O-isobutyryl-dopamine hydrochloride | myristyl alcohol | ethyl oleate |

(cont'd)

Table 2 (cont'd)

| Test Ex. | Sample lot | Sample No. | Ingredient | | |
|---|---|---|---|---|---|
| | | | Dopamine derivative | Higher alcohol | Unsaturated fatty acid monoglyceride alcohol ester |
| 7 | Comp. | h | 3,4-di-O-isobutyryl-epinine hydrochloride | - | - |
| | Invent. | M | 3,4-di-O-isobutyryl-epinine hydrochloride | lauryl alcohol | - |
| 8 | Comp. | i | 3,4-di-O-nicotinoyl-dopaimine hydrochloride | - | - |
| | Invent. | N | 3,4-di-O-nicotinoyl-dopaimine hydrochloride | lauryl alcohol | - |

(ii) Test method

The abdominal hair of a male Wister rat, weighing 170 to 200 g and aged 7 weeks, was removed under anesthesia. After 24 hours, the animal was anesthetized and was confirmed to have no injury in the abdominal skin thereof. Next, the whole abdominal skin was cleansed with 70% ethanol and then peeled off under anesthesia.

The peeled skin was fixed on the lid of a horizontal membrane type in vitro diffusion cell (penetration area: 7.54 $cm^2$) in such a manner that the epidermis served as the donor face. As a receiver solution, 50 ml of physiological saline solution or a 30% aqueous solution of polyethylene glycol 400 was employed. On the donor side, 0.5 g of a solution of each sample was applied by adding dropwise. Then the lid was fixed in a receptor while paying attention to leave no bubbles on the dermal side, followed by storing it in an incubator maintained at 32°C. The receptor solution was stirred with a stirrer and 0.5 ml portions thereof were collected over a period of time so as to determine the amount of dopamine derivative contained therein. The determination was effected by high performance liquid chromatography.

(iii) Test Results

Fig. 1 shows the results of an in vitro test on skin permeability with the use of samples specified in the column of Test Example 1 in Table 1 (namely, comparative samples a and b and invention samples A and B).

As Fig. 1 clearly shows, the skin permeabilities of 3,4-di-O-pivaloyldopamine hydrochloride and 3,4-di-O-isobutyldopamine hydrochloride, which are dopamine derivatives obtained by acylating the catechol group of dopamine, were extremely elevated by adding a lactic acid ester.

Fig. 2 shows the results of an in vitro test on skin permeability with the use of samples specified in the column of Test Example 2 in Table 1 (namely, comparative sample c and invention sample C).

As Fig. 2 clearly shows, the skin permeability of 3,4-di-O-isobutyrylepinine hydrochloride, which is a dopamine derivative obtained by acylating the catechol group of dopamine and substituting a hydrogen in the amino group in the side chain with a methyl group, was extremely elevated by adding a lactic acid ester.

Fig. 3 shows the results of an in vitro test on skin permeability with the use of samples specified in the column of Test Example 3 in Table 1 (namely, comparative sample d and invention sample E).

As Fig. 3 clearly shows, the skin permeability of 3,4-di-O-nicotinoyldopamine hydrochloride, which is a dopamine derivative obtained by acylating the catechol group of dopamine, was extremely elevated by adding a lactic acid ester.

Fig. 4 shows the results of an in vitro test on skin permeability with the use of samples specified in the column of Test Example 4 in Table 1 (namely, comparative sample e and invention sample G).

As Fig. 4 clearly shows, the skin permeability of 3,4-di-O-n-butyryldopamine hydrochloride, which is a dopamine derivative obtained by acylating the catechol group of dopamine, was extremely elevated by adding a lactic acid ester. However, this compound was considerably decomposed after penetrating into the skin.

Figs. 5 to 8 respectively show the results of in vitro tests on skin permeability with the use of samples specified in the columns of Test Examples 5 to 8 in Table 2 (namely, comparative samples f to i and invention samples I and N).

As Figs. 5 to 8 clearly show, the skin permeabilities of 3,4-di-O-pivaloyldopamine hydrochloride, 3,4-di-O-isobutyldopamine hydrochloride, 3,4-di-O-isobutyrylepinine hydrochloride and 3,4-di-O-nicotinoyldopamine hydrochloride were extremely elevated by adding a higher alcohol.

In Test Examples 5 and 6, the invention samples J and L which were prepared by adding high alcohols to the above-mentioned dopamine derivatives and further adding unsaturated fatty acid monohydric alcohol esters added to the mixtures showed the increased skin permeabilities compared with the cases wherein the higher alcohols alone were added.

(2) Test on primary skin irritativeness

(i) Preparation of sample

Samples j and k:

50 mg of dopamine hydrochloride or dobutamine hydrochloride as specified in Table 3 was weighed and 100 mg of propylene glycol and 850 mg of polyethylene glycol were added thereto to obtain a solution containing 5% by weight of dopamine or dobutamine. These preparation were referred to as samples j and k, respectively.

Samples O, Q, U and V:

50 mg of each dopamine derivative hydrochloride as specified in Table 3 was weighed and 50 mg of a lactic acid ester was added thereto. Further, 100 mg of propylene glycol and 800 mg of polyethylene glycol were added thereto to obtain a solution containing 5% by weight of each dopamine derivative hydrochloride was obtained. These solutions were referred to as samples O, Q, U and V, respectively.

Samples P, S, W and X:

50 mg of each dopamine derivative hydrochloride as specified in Table 3 was weighed and 50 mg of a lactic acid ester and 100 mg of an unsaturated fatty acid monohydric alcohol ester, as specified in Table 3, were added thereto. Further, 100 mg of propylene glycol and 700 mg of polyethylene glycol were added thereto to obtain a solution containing 5% by weight of each dopamine derivative hydrochloride. These solutions were referred to as samples P, S, W and X, respectively.

Table 3

| Test Ex. | Sample No. | Ingredient | | |
|---|---|---|---|---|
| | | Dopamine derivative | lactic acid ester | Unsaturated fatty acid monoglyceride alcohol ester |
| 9 | j | dopamine hydrochloride | - | - |
| | k | dobutamine hydrochloride | - | - |
| | O | 3,4-di-O-isobutyryl-epinine hydrochloride | myristyl lactate | - |
| | P | 3,4-di-O-isobutyryl-epinine hydrochloride | myristyl lactate | ethyl linoleate |
| 10 | Q | 3,4-di-O-isobutyryl-dopamine hydrochloride | myristyl lactate | - |
| | S | 3,4-di-O-isobutyryl-dopamine hydrochloride | myristyl lactate | methyl linoleate |

(cont'd)

Table 3 (cont'd)

| Test Ex. | Sample No. | Ingredient: Dopamine derivative | Ingredient: Lactic acid ester | Ingredient: Unsaturated fatty acid monoglyceride alcohol ester |
|---|---|---|---|---|
| 11 | U | 3,4-di-O-nicotinoyl-dopamine hydrochloride | myristyl lactate | - |
| | V | 3,4-di-O-n-butyryl-dopamine hydrochloride | cetyl lactate | - |
| | W | 3,4-di-O-nicotinoyl-dopamine hydrochloride | myristyl lactate | ethyl oleate |
| | X | 3,4-di-O-n-butyryl-dopamine hydrochloride | cetyl lactate | methyl oleate |

(ii) Test method

On the previous day of the test, the dorsal hair of a rabbit was removed. On the day of the test, 2 sites (2.5 cm x 2.5 cm) were located in the both sides of the median line, namely, 4 sites in total. The horny layers of epidermis of two of these sites were injured with an injection needle of size 0.65 mm (236) outer diameter in such a manner as to form parallel crosses, while the other two sites remained uninjured. 5 g of each sample prepared in the above-mentioned manner was applied onto doubled gauge pieces and this was then brought into contact with the injured and uninjured sites. Separately, two pairs of gauge pieces, to which a sample of the same composition as the one described above except that no dopamine derivative was contained had been applied, were brought into contact with the remaining skin of said animal. The back of the rabbit was coated with a linseed oil paper sheet and it was fixed with a tape. Further, it was covered with an elastic net so as to prevent the gauge from sliding. After 24 hours, the gauge pieces were removed and the skin was washed with warm water. Then the conditions of the skin were observed. The observation was repeated after 48 hours and thus the skin conditions were scored in accordance with Drays' skin reaction evaluation table (cf. Table 4). The scores of the uninjured and injured sites after 24 and 72 hours were totaled respectively and the average of each group, having 5 rabbits, was calculated. The obtained value was referred to as the primary skin irritation index.

Table 4

| Skin reaction | Score |
|---|---|
| (1) Formation of erythema and crusta: | |
| No erythema | 0 |
| Slight erythema | 1 |
| Obvious erythema | 2 |
| Moderate to serious erythema | 3 |
| Serious erythema accompanied by crusta | 4 |
| (2) Formation of edema: | |
| No edema | 0 |
| Slight edema | 1 |
| Slight edema with obvious bosselation | 2 |
| Moderate edema with bosselation (ca. 1mm) | 3 |
| Serious edema with bosselation (> ca. 1mm) | 4 |

| Evaluation of skin irritativeness | Total of (1) and (2) |
|---|---|
| Mild | ≦ 2 |
| Moderate | 2-5 |
| Serious | ≧ 5 |

(iii) Results

Table 5 shows the results of a primary skin irritativeness test on the samples shown in the column of Test Example 9 in Table 3 (namely, samples j, k, O and P).

Table 5

| Sample No. | Primary skin irritation index |
| --- | --- |
| j | 3.5 |
| k | 3.5 |
| O | 3.5 |
| P | 1.4 |

As Table 5 clearly shows, the sample P, which contained ethyl linoleate as an unsaturated fatty acid monohydric alcohol ester, showed an extremely reduced skin irritativeness, compared with the samples j, k and O.

Table 6 shows the results of a primary skin irritativeness test on the samples shown in the column of Test Example 10 in Table 3 (namely, samples Q and S).

Table 6

| Sample No. | Primary skin irritation index |
| --- | --- |
| Q | 3.5 |
| S | 1.3 |

As Table 6 clearly shows, the sample S, which was prepared by adding methyl linoleate to the samples Q, showed an extremely reduced skin irritativeness, compared with the sample Q.

Table 7 shows the results of a primary skin irritativeness test on the samples shown in the column of Test Example 11 in Table 3 (namely, samples U, V, W and X).

Table 7

| Sample No. | Primary skin irritation index |
|---|---|
| U | 3.5 |
| V | 3.5 |
| W | 2.5 |
| X | 2.0 |

As Table 7 clearly shows, the samples W and X, which were respectively prepared by adding ethyl oleate and methyl oleate to the samples U and V, showed each an extremely reduced skin irritativeness, compared with the samples U and V.

To further illustrate the present invention, the following Synthesis Examples and Examples will be given.

SYNTHESIS EXAMPLE 1

(1) Synthesis of N-carbobenzoxydopamine

38.2 g (100 mmol) of sodium borate and 18.9 g (100 mmol) of dopamine hydrochloride were dissolved in 250 ml of water. To the solution thus obtained, was added a 2 N sodium hydroxide solution under a nitrogen gas stream to adjust the pH value to 9. Then 17.1 g (100 mmol) of benzyloxycarbonyl chloride was added dropwise thereto while stirring at 15°C for 4 hours. Simultaneously, a 2 N sodium hydroxide solution was added to maintain the pH value of the reaction mixture to 9. After continuing the stirring for 2 hours, the mixture was adjusted to pH 1 to 2 with hydrochloric acid and then extracted with diethyl ether. Next, it was dried over anhydrous magnesium sulfate and the solvent was distilled off. After washing the residue with petroleum ether, the crude crystals thus obtained were recrystallized from ethanol/n-hexane (1: 1 by volume). Thus 27.6 g (yield: 96.5%) of the desired compound (m.p.: 134-135°C) was obtained in the form of white crystals.

$^1$H-NMR (solvent: $CD_3OD$, internal standard: TMS):

δppm: 2.63 (2H, t, J = 6.2 Hz, $PhCH_2$), 3.28 (2H, t, J = 7.2 Hz, $CH_2N$), 5.06 (2H, s, $OCH_2Ph$), 6.50 (1H, m, Ar), 6.68 (2H, m, Ar) and 7.31 (5H, m, Ar).

(2) Synthesis of 3,4-di-O-pivaloyl-N-carbobenzoxydopamine

To 150 ml of a pyridine solution containing 23.0 g (98 mmol) of N-carbobenzoxydopamine obtained in Synthesis Example 1-(1), was added 24.1 g (200 mmol) of pivaloyl chloride dropwise at 5 to 10°C. After stirring at room temperature for 12 hours, the mixture was allowed to react at 35 to 40°C for 3 hours. Further, the reaction mixture was poured into ice and a sodium carbonate solution and the stirring was continued at room temperature for 30 minutes. After extracting with diethyl ether and drying over anhydrous magnesium sulfate, the solvent was distilled off to give crude crystals. The resulting crude crystals then recrystallized from petroleum ether to obtain 27.1 g (yield: 80.1%) of the desired compound in the form of white crystals (m.p.: 50-51°C).

$^1$H-NMR (solvent: $CDCl_3$, internal standard: TMS):

δppm: 1.32 (18H, s, $CH_3$), 2.79 (2H, t, J = 6.8 Hz, $PhCH_2$), 3.42 (2H, t, J = 6.6 Hz, $CH_2N$), 5.10 (2H, s, $OCH_2Ph$), 6.95 (1H, s, Ar), 7.03 (2H, s, Ar) and 7.34 (5H, m, Ar).

(3) Synthesis of 3,4-di-O-pivaloyldopamine hydrochloride

5.0 g (11 mmol) of the 3,4-di-O-pivaloyl-N-carbobenzoxydopamine obtained in Synthetic Example 1-(2) was dissolved in 50 ml of methanol and subjected to catalytic reduction under atmospheric pressure in the presence of palladium carbon for 6 hours. After filtering off the catalyst, a hydrogen chloride gas was bubbled thereto under ice-cooling for 10 minutes. Then the solvent was distilled off under reduced pressure to obtain 3.7 g (yield: 94.1%) of the desired compound in the form of hygroscopic crystals.
$^1$H-NMR (solvent: $CDCl_3$, internal standard: TMS):
δppm: 1.33 (18H, s, $CH_3$), 2.99 (2H, m, $PhCH_2$), 3.20 (2H, m, $CH_2N$) and 7.13-7.24 (3H, m, Ar).

SYNTHESIS EXAMPLE 2

(1) Synthesis of 3,4-di-O-isobutyryl-N-carbobenzoxydopamine

To 80 ml of a pyridine solution containing 10.7 g (37 mmol) of N-carbobenzoxydopamine obtained in Synthesis Example 1-(1) was added dropwise 9.9 g (93 mmol) of isobutyryl chloride at 5 to 10°C. After stirring at room temperature for 12 hours, the mixture was allowed to react at 35 to 40°C for 3 hours. Then, the reaction mixture was poured into ice and a sodium carbonate solution and stirred at room temperature for 30 minutes. The mixture was extracted with diethyl ether and the resulting extract was dried over anhydrous magnesium sulfate. The solvent was distilled and the thus-obtained crude crystals were recrystallized from diisopropyl ether/petroleum ether (1: 2 by volume) to obtain 11.9 g (yield: 74.7%) of the desired compound in the form of white cyrstals (m.p. 44-46°C).
$^1$H-NMR (solvent: $CDCl_3$, internal standard: TMS):
δppm: 1.29 (12H, d, J = 7.0 Hz, $CH_3$), 2.77 (2H, m, >CH), 2.81 (2H, broad, s, $PhCH_2$), 3.43 (2H, m, $CH_2N$), 5.10 (2H, s, $OCH_2Ph$), 6.99-7.09 (3H, m, Ar), 7.34 (5H, m, Ar).

(2) Synthesis of 3,4-di-O-isobutyryldopamine hydrochloride

7.0 g (16.4 mmol) of 3,4-di-O-isobutyryl-N-carbobenzoxydopamine obtained in Synthesis Example 2-(1) was dissolved in 50 ml of methanol and the mixture was subjected to catalytic reduction under atmospheric pressure in the presence of palladium carbon for 6 hours. After filtering the catalyst off, a hydrogen chloride gas was bubbled thereto under ice-cooling for 10 minutes. The solvent was distilled under reduced pressure to obtain 4.4 g (yield: 81.4%) of the desired compound in the form of hygroscopic crystals.
$^1$H-NMR (solvent: $CDCl_3$, internal standard: TMS):
δppm: 1.28 (12H, d, J = 7.0 Hz, $CH_3$), 2.75 (2H, m, >CH), 2.92 (2H, m, $PhCH_2$), 3.15 (2H, m, $CH_2N$), 7.00-7.09 (3H, m, Ar).

SYNTHESIS EXAMPLE 3

Synthesis of 3,4-di-O-isobutyrylepinine hydrochloride

14.0 g (57 mmol) of epinine hydrobromide and 21.4 g (0.2 mol) of isobutyryl hydrochloride were added to 50 ml of 1,4-dioxane containing anhydrous hydrogen chloride in a concentration of 10%. The mixture was allowed to react at 70°C for 12 hours and then the reaction mixture was concentrated under reduced pressure. The thus-obtained residue was dissolved in a 10% aqueous solution of sodium hydrogencarbonate so as to make the pH value 8. Then, it was extracted with chloroform and the resulting extract was dried over anhydrous magnesium sulfate. After removing the anhydrous magnesium sulfate by filtration, a hydrogen chloride gas was bubbled into the filtrate under ice-cooling. The solvent was distilled to obtain crude crystals. The thus-obtained crystals were recrystallized from ethyl acetate to obtain 17.8 g (yield: 91.7%) of the desired compound in the form of while crystals (m.p. 127-128°C).
$^1$H-NMR (solvent: $CDCl_3$, internal standard: TMS):
δppm: 1.29 (12H, d, J = 7.0 Hz, $CH_3$), 2.69 (3H, t, J = 5.4 Hz, $NCH_3$), 2.76 (2H, m, >CH), 3.17-3.25 (4H, m, $PhCH_2CH_2N$), 7.09-7.15 (3H, m, Ar).

SYNTHESIS EXAMPLE 4

(1) Synthesis of 3,4-di-O-nicotinoyl-N-carbobenzoxydopamine

19.0 g (66 mmol) of N-carbobenzoxydopamine obtained in Synthesis Example 1-(1) and 37.2 g (209 mmol) of hydrochloride of nicotinoyl chloride were added to 200 ml of pyridine. After stirring at room temperature for 12 hours, the mixture was allowed to react at 35-40°C for 2 hours. Then, the reaction mixture was poured into ice and a sodium carbonate solution followed by extraction with chloroform. The resulting extract was dried over anhydrous magnesium sulfate and the solvent was distilled off to obtain crude crystals. The thus-obtained crystals were recrystallized from ethyl acetate/diethyl ether (1: 5 by volume) to obtain 23.5 g (yield: 71.6%) of the desired compound in the form of white crystals (m.p. 77-78°C).
$^1$H-NMR (solvent: $CDCl_3$, internal standard: TMS):
δppm: 2.90 (2H, m, $PhCH_2$), 3.50 (2H, m, $CH_2N$), 5.11 (2H, s, $OCH_2Ph$), 7.23-7.37 (10H, m, Ar), 8.29 (2H, m, Ar), 8.77 (2H, m, Ar), 9.24 (2H, d, J = 1.9 Hz, Ar)

(2) Synthesis of 3,4-di-O-nicotinoyldopamine hydrochloride

10.0 g (20 mmol) of 3,4-di-O-nicotinoyl-N-carbobenzoxydopamine obtained in Synthesis Example 4-(1) was added to 200 ml of trifluoroacetic acid and heated under reflux for 1 hour. After distilling the solvent under reduced pressure, the residue was dissolved in 1,4-dioxane and a hydrogen chloride gas was bubbled thereto under ice cooling. Then, the solvent was distilled under reduced pressure and the thus-obtained crude crystals were recrystallized from methanol/ethyl acetate (1: 3 by volume) to obtain 4.4 g (yield: 46%) of the desired compound in the form of white crystals (m.p. 169-172°C).
$^1$H-NMR (solvent: $CD_3OD$, internal standard: TMS):
δppm: 3.12 (2H, m, $PhCH_2$), 3.29 (2H, m, $CH_2N$), 7.45-7.60 (3H, m, Ar), 8.23 (2H, m, Ar), 9.11 (2H, m, Ar), 9.17 (2H, m, Ar), 9.51 (2H, m, Ar)

EXAMPLE 1

Oily ointment

An oily ointment having the following composition was prepared as described below.

Cetyl lactate was heated to 60°C and 3,4-di-O-isobutyryldopamine hydrochloride and ethylenediamine tetraacetic acid disodium salt were added thereto. The resulting mixture was homogenized by stirring. Separately, sorbitan oleate, white vaseline and ethyl linoleate were heated to 60°C and homogeneously mixed together, and to this mixture was added the above-obtained mixture. The whole mixture was then thoroughly stirred and cooled to room temperature to give a desired oily ointment.

| | [% by weight] |
|---|---|
| 3,4-di-O-isobutyryldopamine hydrochloride | 5.0 |
| ethylenediamine tetraacetic acid disodium salt | 0.5 |
| cetyl lactate | 3.0 |
| sorbitan oleate | 3.0 |
| white petroleum jelly | 86.5 |
| ethyl linoleate | 2.0 |

EXAMPLE 2

Hydrophilic ointment

A hydrophilic ointment having the following composition was prepared as described below.

Myristyl lactate, ethylenediaminetetraacetic acid disodium salt, polyoxyethylene (20) sorbitan monooleate, official macrogol ointment (J.P.) and ethyl benzoate as a preservative were heated to 60°C and homogeneously mixed together. Then a fine powder of 3,4-di-O-isobutyryldopamine hydrochloride and methyl linoleate were added thereto. The obtained mixture was thoroughly mingled under cooling to give a desired hydrophilic ointment.

| | [% by weight] |
|---|---|
| 3,4-di-O-isobutyryldopamine hydrochloride | 5.0 |
| myristyl lactate | 5.0 |
| ethylenediamine tetraacetic acid disodium salt | 0.5 |
| polyoxyethylene (20) sorbitan monooleate | 2.0 |
| macrogol ointment (JP) | 82.4 |
| ethyl benzoate | 0.1 |
| methyl linoleate | 5.0 |

EXAMPLE 3

O/W type cream

An O/W type cream having the following composition was prepared as described below.

Squalane, isopropyl myristate, stearic acid, stearic acid monoglyceride, sorbitan monopalmitate, polyoxyethylene (20) sorbitan monostearate, myristyl lactate and ethylenediamine tetraacetic acid disodium salt were heated to 70°C and homogeneously molten together. Then 3,4-di-O-isobutyrylepinine hydrochloride was added thereto and the obtained mixture was homogenized by stirring. Separately, propylene glycol, benzoic acid as an antiseptic and purified water were heated to 60°C and then slowly added thereto. The mixture was cooled to approximately 30°C under stirring. Thus a desired oil-in-water type of cream was obtained.

| | [% by weight] |
|---|---|
| 3,4-di-O-isobutyrylepinine hydrochloride | 5.0 |
| myristyl lactate | 5.0 |
| squalane | 8.0 |
| isopropyl myristate | 4.0 |
| stearic acid | 4.0 |
| stearic acid monoglyceride | 4.0 |
| sorbitan monopalmitate | 1.5 |
| polyoxyethylene (20) sorbitan monostearate | 1.5 |
| ethylenediaminetetraacetic acid disodium salt | 0.5 |
| propylene glycol | 5.0 |
| benzoic acid | 0.1 |
| purified water | 61.4 |

EXAMPLE 4

Oily ointment

An oily ointment having the following composition was prepared as described below.

Cetyl lactate and lauryl lactate were heated to 55°C and ethylenediaminetetraacetic acid disodium salt and 3,4-di-O-isobutyrylepinine hydrochloride were added thereto. The obtained mixture was homogenized. Separately, the another mixture was prepared by heating octyldodecyl myristate, sorbitan sesqui-oleate, purified lanolin, propylene glycol monostearate and white vaseline to 70°C and homogenizing them. The former mixture was added to the latter one and the resulting mixture was cooled under stirring to give an oily ointment.

| | [% by weight] |
|---|---|
| 3,4-di-O-isobutyrylepinine hydrochloride | 5.0 |
| cetyl lactate | 3.0 |
| lauryl lactate | 3.0 |
| octyldodecyl myristate | 10.0 |
| sorbitan sesqui-oleate | 5.0 |
| purified lanolin | 10.0 |
| propylene glycol monostearate | 2.0 |
| ethylenediaminetetraacetic acid disodium salt | 0.5 |
| white petroleum jelly | 61.5 |

EXAMPLE 5

Oily ointment base

An oily ointment base having the following composition was prepared as described below.

Propylene glycol was heated to 60°C and 3,4-di-O-nicotinoyldopamine hydrochloride and ethylenediaminetetraacetic acid disodium salt were added thereto. These were mixed by stirring. Then a mixture of cetyl lactate and PLASTIBASE (registered trade name of the product manufactured by ER Squib & Sons Inc., Japan Co.; comprising 95% by weight of liquid paraffin and 5% by weight of polyethylene having a molecular weight of 10,000 to 30,000), which had been preliminarily heated to 60°C and mixed, was added thereto. The resulting mixture was cooled to room temperature under stirring to give a desired oily ointment base.

| | [% by weight] |
|---|---|
| 3,4-di-O-nicotinoyldopamine hydrochloride | 5.0 |
| cetyl lactate | 10.0 |
| propylene glycol | 15.0 |
| ethylenediaminetetraacetic acid disodium salt | 0.5 |
| PLASTIBASE | 69.5 |

EXAMPLE 6

Plaster

A plaster having the following composition was prepared as described above.

SIS rubber (manufactured by Shell Chemical Co.), ARKON P-100 (registered trade name of the product manufactured by Arakawa Kagaku Kogyo K.K., alicyclic petroleum resin) and liquid paraffin, which had been molten at 130°C, were mixed with cetyl lactate, glycerol monooleate and dibutylhydroxytoluene, which had been molten at 70°C, and the mixture was cooled to 60°C under stirring. Next, 3,4-di-O-isobutyryldopamine hydrochloride and ethylenediaminetetraacetic acid disodium salt were added thereto and the resulting mixture was homogenized by stirring. Then it was spreaded onto laminated films of polyethylene terephthalate to give a desired plaster.

| | [% by weight] |
|---|---|
| SIS rubber | 35.0 |
| ARKON P-100 | 24.0 |
| liquid paraffin | 24.5 |
| 3,4-di-O-isobutyryldopamine hydrochloride | 5.0 |
| cetyl lactate | 5.0 |
| glycerol monooleate | 5.0 |
| dibutylhydroxytoluene | 1.0 |
| ethylenediaminetetraacetic acid disodium salt | 0.5 |

EXAMPLE 7

Plaster

A plaster having the following composition was prepared as desired below.

SIS rubber (manufactured by Shell Chemical Co.), ARKON P-100 (registered trade name of the product manufactured by Arakawa Kagaku Kogyo K.K., alicyclic petroleum resin) and liquid paraffin, which had been molten at 130°C, were mixed with lauryl alcohol and dibutylhydroxytoluene, which had been molten at 70°C, and the mixture was cooled to 60°C under stirring. Next, 3,4-di-O-pivaloyldopamine hydrochloride, methyl linoleate and ethylenediaminetetraacetic acid disodium salt were added thereto and the resulting mixture was homogenized by stirring. Then it was spread onto laminated films of polyethylene vinyl alcohol to give a desired plaster.

| | [% by weight] |
|---|---|
| SIS rubber | 35.0 |
| ARKON P-100 | 24.0 |
| liquid paraffin | 24.5 |
| 3,4-di-O-pivaloyldopamine hydrochloride | 5.0 |
| lauryl alcohol | 5.0 |
| methyl linoleate | 5.0 |
| dibutylhydroxytoluene | 1.0 |
| ethylenediaminetetraacetic acid disodium salt | 0.5 |

**Claims**

1. A percutaneously absorbable pharmaceutical preparation which comprises:
   (A) a diacylated dopamine derivative represented by the formula (I):

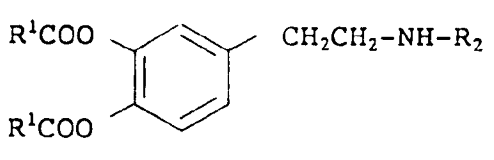

(I)

   wherein each $R^1$ represents an alkyl group having 3 to 7 carbon atoms, a cycloalkyl group having 3 to 6 carbon atoms, a substituted or unsubstituted phenyl group or a substituted or unsubstituted nitrogen-containing heterocylic group; and $R^2$ represents a hydrogen atom or an alkyl group having up to 4 carbon atoms; or a salt thereof; and
   (B) one or more compounds selected from lactic acid esters selected from myristyl lactate, cetyl lactate and lauryl lactate, and a saturated or unsaturated alcohol having 12 to 18 carbon atoms.

2. A preparation as claimed in Claim 1, wherein $R^1$ is a branched alkyl group.

3. A preparation claimed in Claim 1, wherein $R^1$ is a pyridyl group.

4. A preparation as claimed in any preceding claim, wherein said diacylated dopamine derivative or a salt thereof is contained in an amount of 1 to 20% by weight based on the weight of the preparation.

5. A preparation as claimed in any preceding claim, wherein said ingredient (B) is contained in a proportion of 0.01 to 5 parts by weight to one part of the dopamine derivative or salt thereof.

6. A preparation as claimed in any preceding claim which also contains (C) an unsaturated fatty acid monohydric alcohol ester.

7. A preparation as claimed in Claim 6, wherein said unsaturated fatty acid monohydric alcohol ester is methyl linoleate, ethyl linoleate, propyl linoleate, methyl oleate, ethyl oleate or methyl linolenate.

8. A preparation as claimed in Claim 6 or 7, wherein said unsaturated fatty acid monohydric alcohol ester (C) is contained in a proportion of 0.1 to 10 parts by weight to one part of the dopamine derivative or salt thereof.

9. Use of a compound of formula (I) as defined in Claim 1 or a salt thereof in the manufacture of a percutaneous preparation, as defined in any preceding claim.

**Patentansprüche**

1. Perkutan absorbierbare pharmazeutische Zusammensetzung, welche umfaßt:
(A) ein diacyliertes Dopamin-Derivat, dargestellt durch die Formel (I):

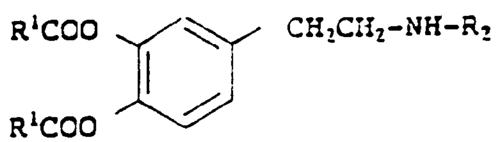

(I)

worin $R^1$ jeweils eine Alkylgruppe mit 3 bis 7 Kohlenstoffatomen, eine Cycloalkylgruppe mit 3 bis 6 Kohlenstoffatomen, eine substituierte oder unsubstituierte Phenylgruppe oder eine substituierte oder unsubstituierte Stickstoff-enthaltende heterozyklische Gruppe bedeutet; und $R^2$ ein Wasserstoffatom oder eine Alkylgruppe mit bis zu 4 Kohlenstoffatomen bedeutet; oder ein Salz davon;
und
(B) eine oder mehrere Verbindungen, ausgewählt aus Milchsäureestern, ausgewählt aus Myristyllactat, Cetyllactat und Lauryllactat, und einem gesättigten oder ungesättigten Alkohol mit 12 bis 18 Kohlenstotfatomen.

2. Zusammensetzung nach Anspruch 1, worin $R^1$ eine verzweigte Alkylgruppe ist.

3. Zusammensetzung nach Anspruch 1, worin $R^1$ eine Pyridylgruppe ist.

4. Zusammensetzung nach einem der vorhergehenden Ansprüche, worin das diacylierte Dopamin-Derivat oder ein Salz davon in einer Menge von 1 bis 20 Gew.-%, bezogen auf das Gewicht der Zusammensetzung, enthalten ist.

5. Zusammensetzung nach einem der vorhergehenden Ansprüche, worin der Bestandteil (B) in einem Verhältnis von 0,01 bis 5 Gew.-Anteilen auf einen Teil des Dopamin-Derivats oder des Salzes davon enthalten ist.

6. Zusammensetzung nach einem der vorhergehenden Ansprüche, die weiterhin (C) einen Ester einer ungesättigten Fettsäure mit einem einwertigen Alkohol enthält.

7. Zusammensetzung nach Anspruch 6, worin der Ester einer ungesättigten Fettsäure mit einem einwertigen Alkohol Methyllinoleat, Ethyllinoleat, Propyllinoleat, Methyloleat, Ethyloleat oder Methyllinolenat ist.

8. Zusammensetzung nach Anspruch 6 oder 7, worin der Ester (C) einer ungesättigten Fettsäure mit einem einwertigen Alkohol in einem Verhältnis von 0,1 bis 10 Gew.-Anteilen auf einen Teil des Dopamin-Derivats

oder des Salzes davon enthalten ist.

9. Verwendung einer Verbindung der Formel (I), wie in Anspruch 1 definiert, oder eines Salzes davon, für die Herstellung einer perkutanen Zusammensetzung, wie in einem der vorhergehenden Ansprüche definiert.

## Revendications

1. Préparation pharmaceutique absorbable par voie percutanée, qui comprend:
(A) un dérivé de dopamine diacylée, représenté par la formule (I):

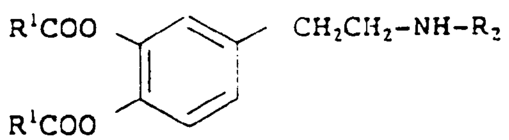

(I)

dans laquelle chaque R1 représente un groupe alkyle ayant 3 à 7 atomes de carbone, un groupe cycloalkyle ayant 3 à 6 atomes de carbone, un groupe phényle substitué ou non substitué ou un groupe hétérocyclique azoté substitué ou non substitué ; et $R^2$ représente un atome d'hydrogène ou un groupe alkyle ayant jusqu'à 4 atomes de carbone ; ou un sel de ce dérivé; et
(B) un ou plusieurs composés, choisi(s) parmi des esters de l'acide lactique eux-mêmes choisis parmi le lactate de myristyle, le lactate de cétyle et le lactate de lauryle, et un alcool, saturé ou insaturé, ayant 12 à 18 atomes de carbone.

2. Préparation telle que revendiquée à la revendication 1, dans laquelle $R^1$ représente un groupe alkyle ramifié.

3. Préparation revendiquée à la revendication 1, dans laquelle $R^1$ repréente un groupe pyrdyle.

4. Préparation telle que revendiquée dans l'une quelconque des revendications précédentes, dans laquelle le dérivé de dopamine diacylé, ou un sel de ce dérivé, est contenu en une quantité de 1 à 20 % en poids, sur la base du poids de la préparation.

5. Préparation telle que revendiquée dans l'une quelconque des revendications précédentes, dans laquelle ledit ingrédient (B) est contenu en une proportion de 0,01 à 5 parties en poids pour 1 partie du dérivé de dopamine ou de son sel.

6. Préparation telle que revendiquée dans l'une quelconque des revendications précédentes, qui contient également (C) un ester d'acide gras insaturé d'un monoalcool.

7. Préparation telle que revendiquée à la revendication 6, dans laquelle l'ester d'acide gras insaturé de monoalcool est le linoléate de méthyle, le linoléate d'éthyle, le linoléate de propyle, l'oléate de méthyle, l'oléate d'éthyle ou le linolénate de méthyle.

8. Préparation telle que revendiquée à la revendication 6 ou 7, dans laquelle l'ester (C) d'acide gras insaturé et d'un monoalcool est contenu en une proportion de 0,1 à 10 parties en poids pour 1 partie du dérivé de la dopamine ou de son sel.

9. Utilisation d'un composé de formule (I), telle que définie à la revendication 1, ou d'un sel de ce composé pour la fabrication d'une préparation à administration percutanée, telle que définie dans l'une quelconque des revendications précédentes.

Fig. 1

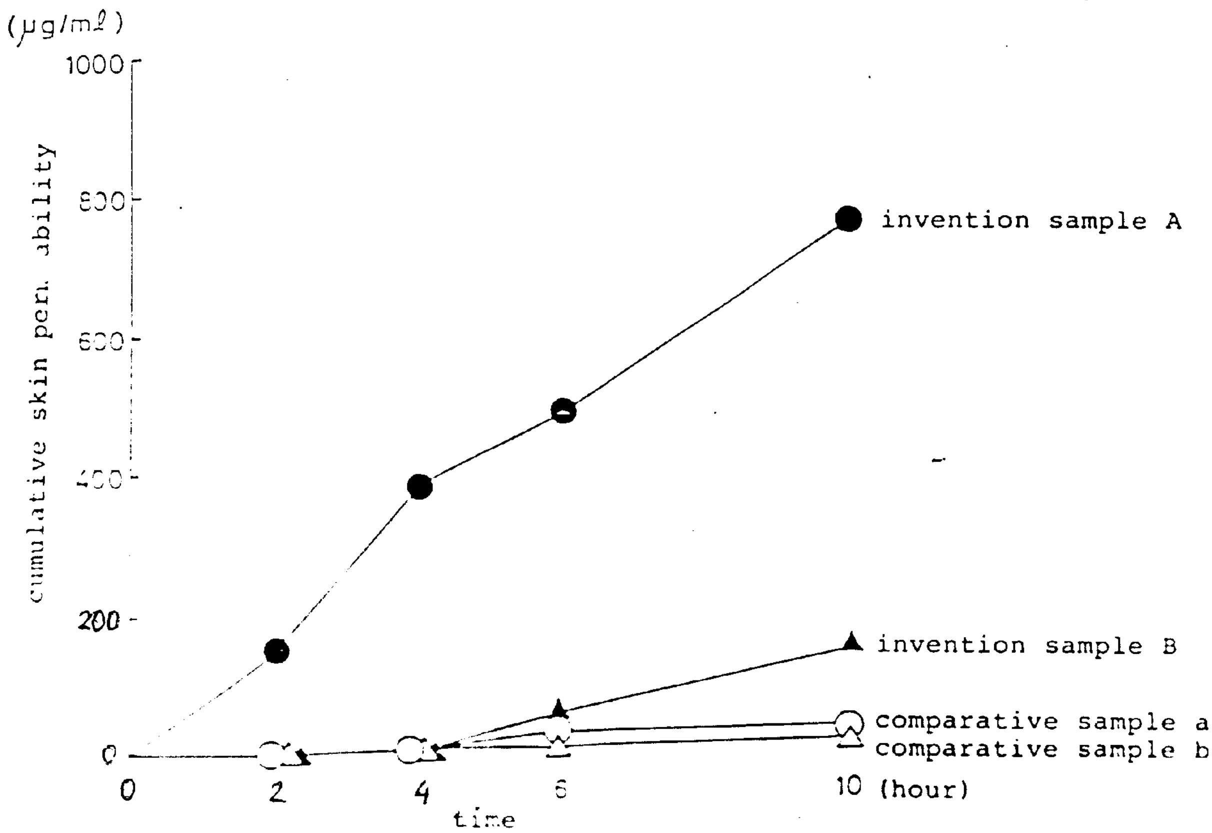
(μg/mℓ)
1000
800
600
400
200
0
cumulative skin pern. ability
invention sample A
invention sample B
comparative sample a
comparative sample b
0
2
4
6
10 (hour)
time

Fig. 2

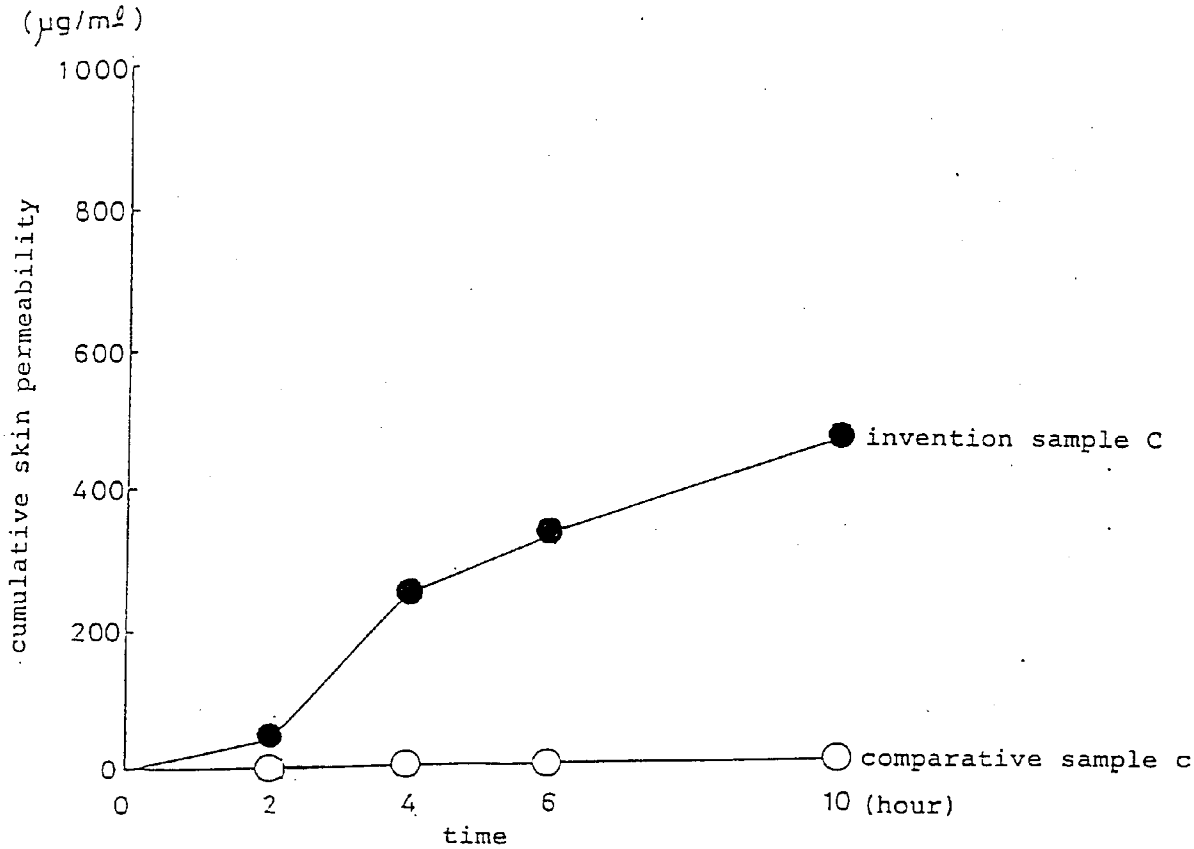
(μg/mℓ)
1000
800
600
400
200
0
cumulative skin permeability
invention sample C
comparative sample c
0
2
4
6
10 (hour)
time

Fig. 3

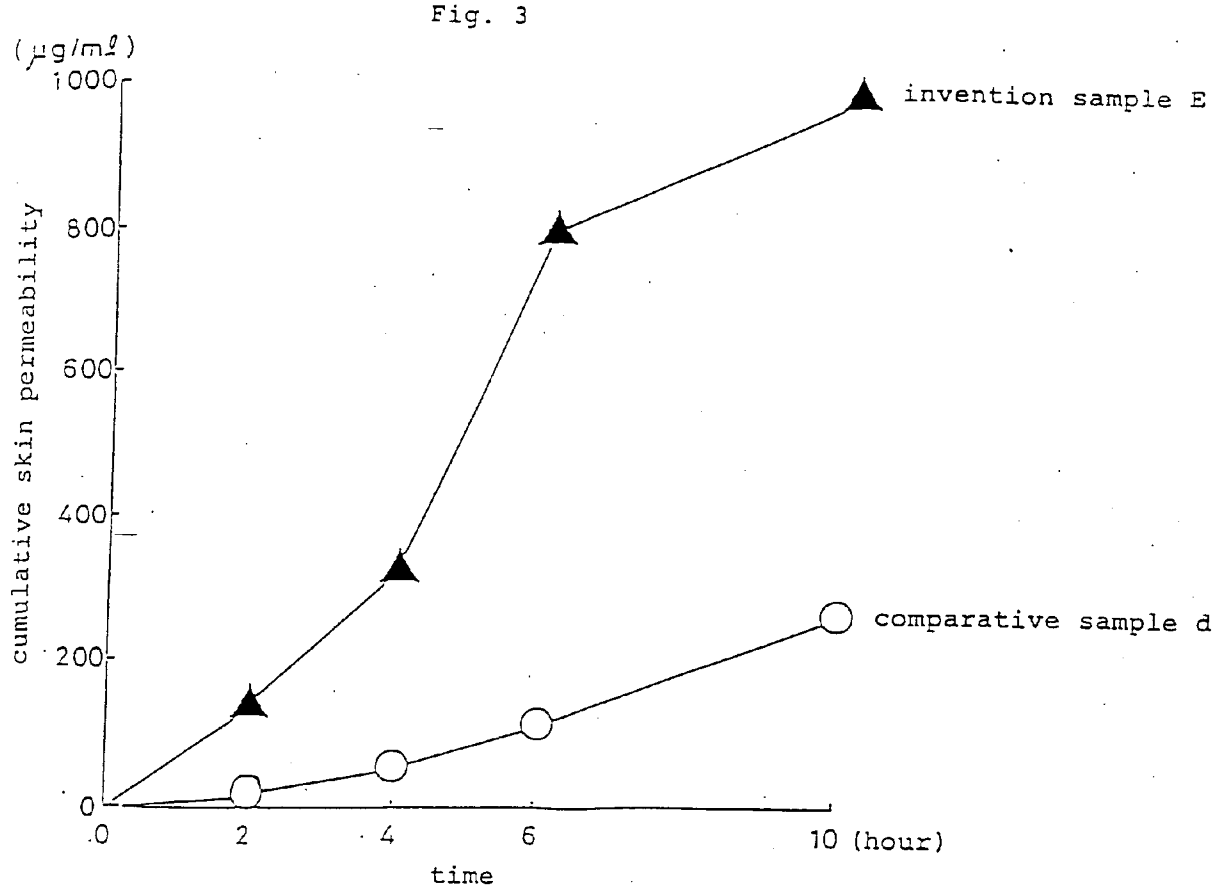
(μg/mℓ)
1000
800
600
400
200
0
cumulative skin permeability
.0
2
4
6
10 (hour)
time
invention sample E
comparative sample d

Fig. 4

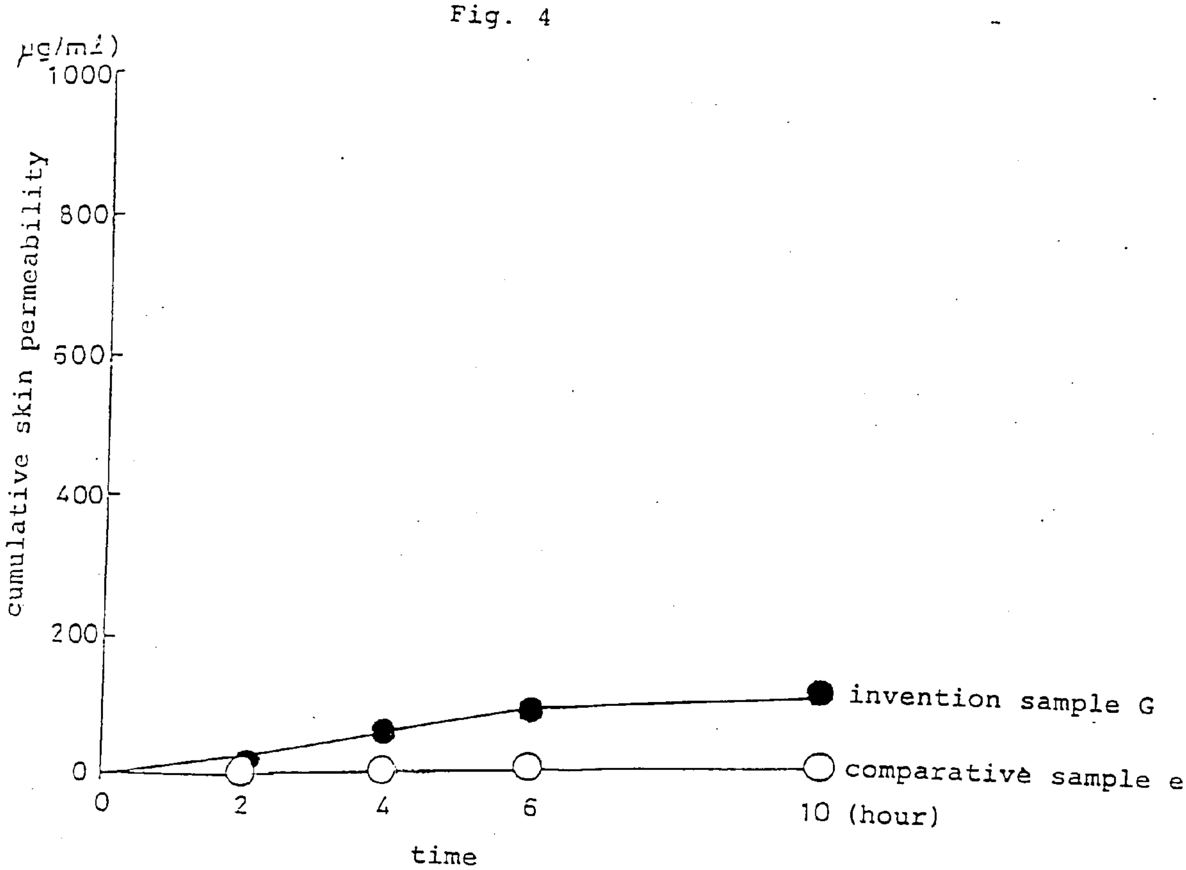
μg/ml)
1000
800
600
400
200
0
cumulative skin permeability
0
2
4
6
10 (hour)
time
invention sample G
comparative sample e

Fig. 5

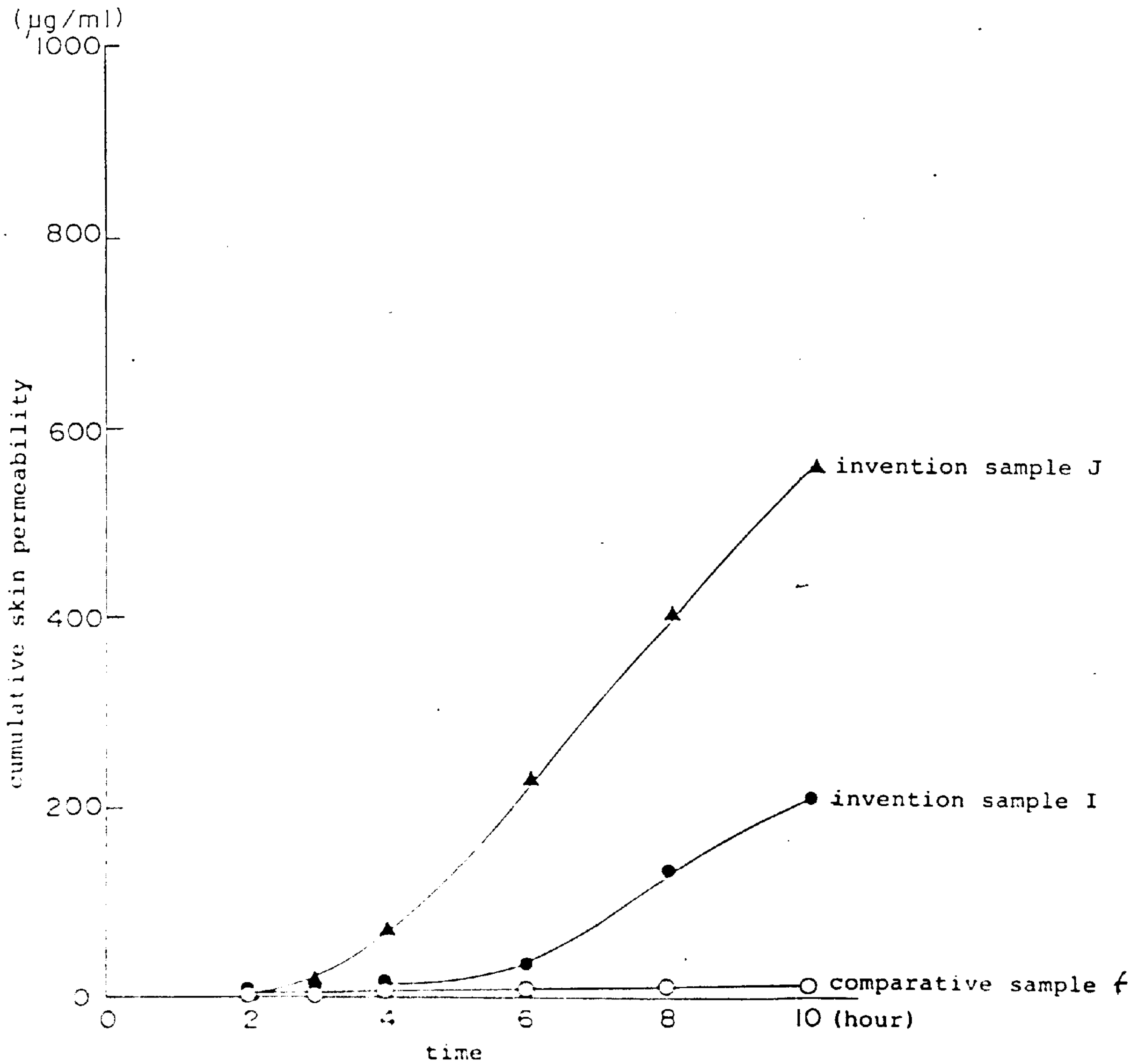
(μg/ml)
1000
800
600
400
200
0
cumulative skin permeability
0
2
4
6
8
10 (hour)
time
invention sample J
invention sample I
comparative sample f

Fig. 6

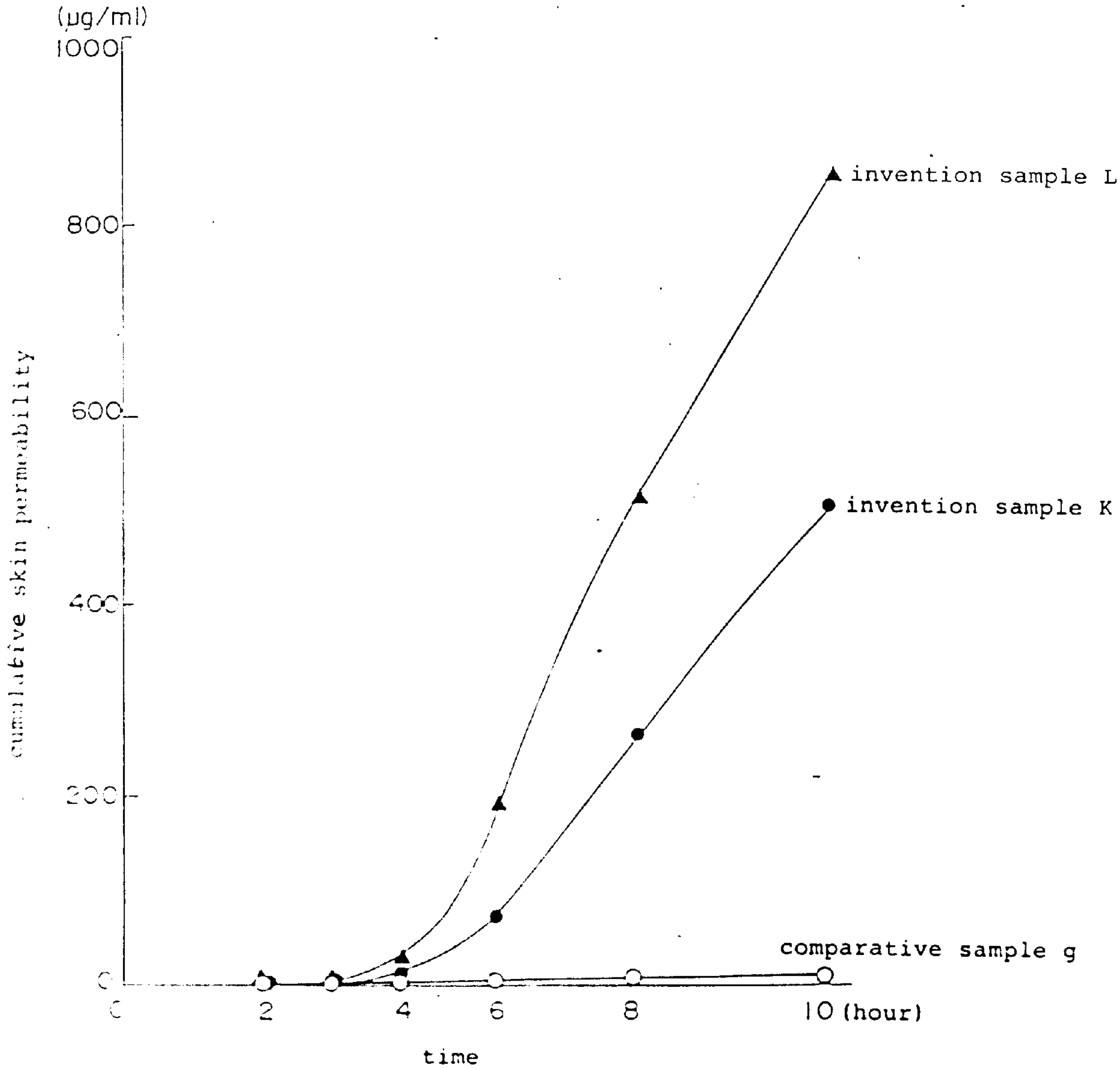
(µg/ml)
1000
800
600
400
200
0
cumulative skin permeability
invention sample L
invention sample K
comparative sample g
0
2
4
6
8
10 (hour)
time

Fig. 7

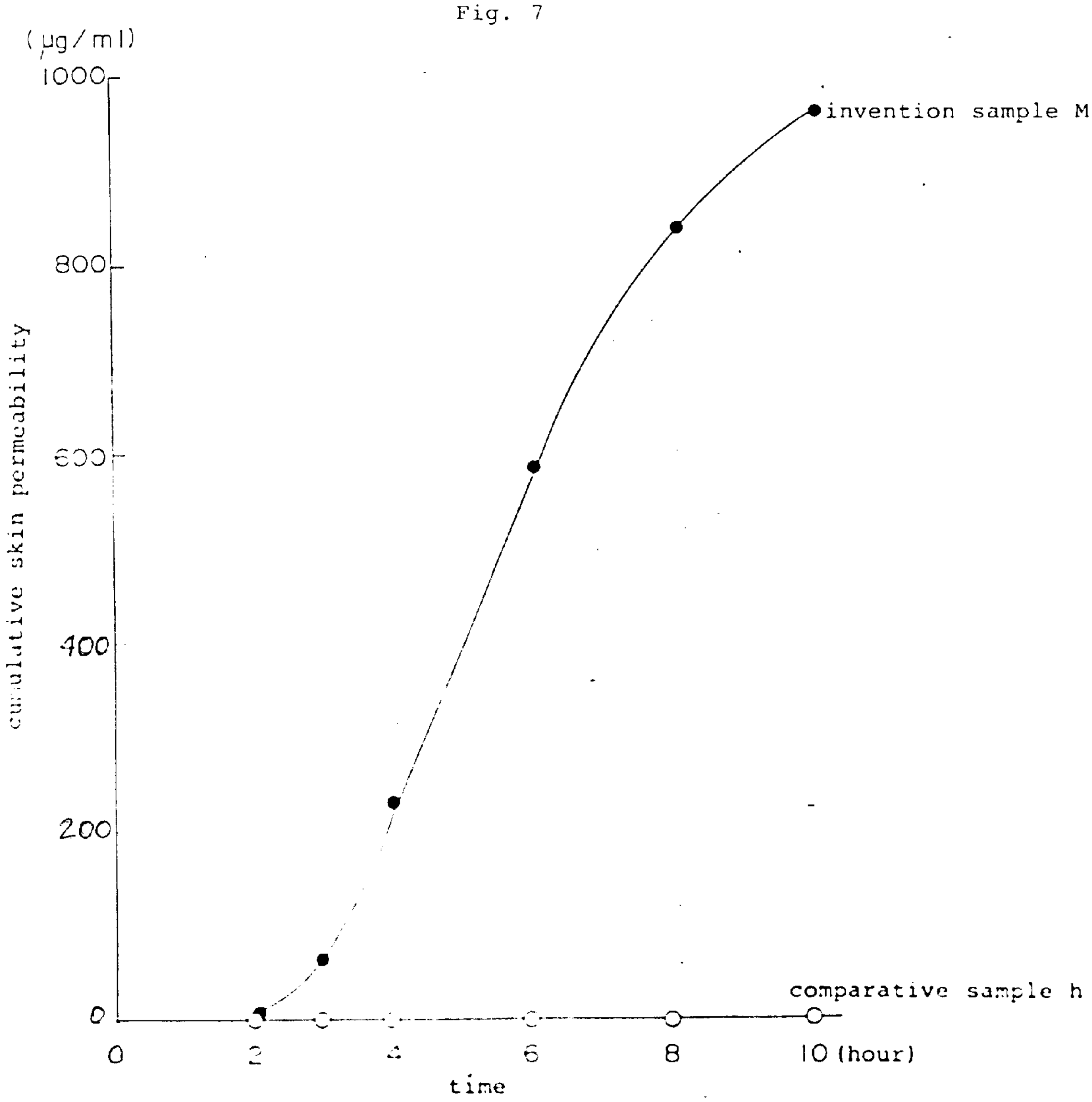
(μg/ml)
1000
800
600
400
200
0
cumulative skin permeability
invention sample M
comparative sample h
0
2
4
6
8
10 (hour)
time

Fig. 8

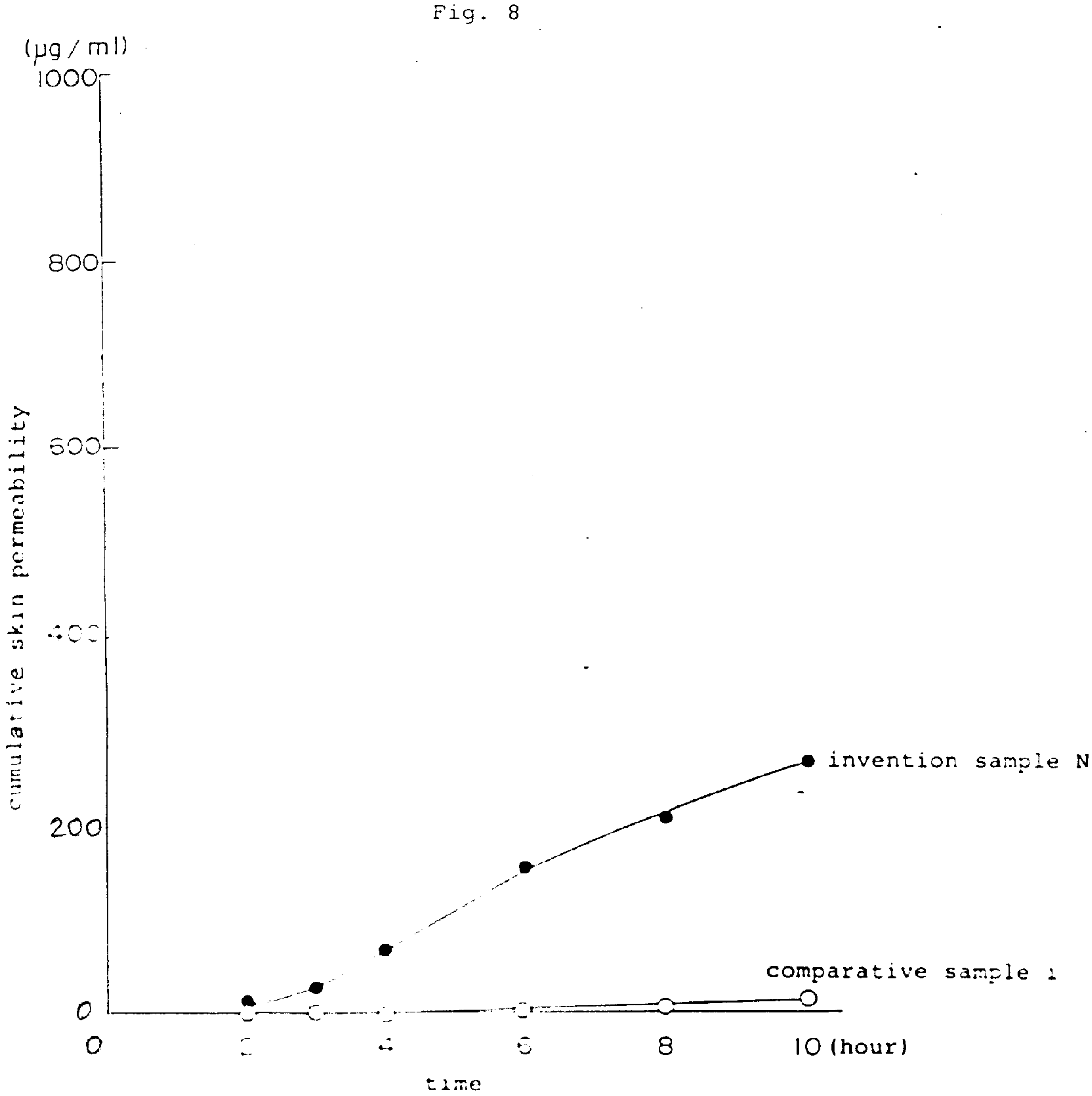
(μg / ml)
1000
800
600
200
0
cumulative skin permeability
invention sample N
comparative sample 1
0
8
10 (hour)
time